**Europäisches Patentamt**

**European Patent Office**

**Office européen des brevets**

(19)

(11) Publication number : **0 425 154 B1**

(12) **EUROPEAN PATENT SPECIFICATION**

(45) Date of publication of patent specification :
15.09.93 Bulletin 93/37

(51) Int. Cl.⁵ : **A61K 9/22,** A61K 9/20,
A61K 9/26, A61K 9/16

(21) Application number : 90311278.7

(22) Date of filing : 16.10.90

(54) **Sustained release elements.**

(30) Priority : 23.10.89 FR 8913818

(43) Date of publication of application :
02.05.91 Bulletin 91/18

(45) Publication of the grant of the patent :
15.09.93 Bulletin 93/37

(84) Designated Contracting States :
BE CH DE DK ES FR GB IT LI NL SE

(56) References cited :
EP-A- 0 277 740
EP-A- 0 280 613
EP-A- 0 281 236
FR-A- 2 160 410
FR-A- 2 309 212
GB-A- 2 122 490

(73) Proprietor : **Dow Corning France S.A.**
**Route des Cretes BP 43 Sophia Antipolis Les**
**Bouillides**
**F-06561 Valbonne (FR)**

(72) Inventor : **Aguadisch, Louis Michel Jaques**
**720 A. Chemin de Villebruc**
**F-06560 Valbonne (FR)**
Inventor : **Grisoni, Bernard Francis**
**21506 I Lake Forest Drive**
**El Toro, California 92630 (US)**
Inventor : **Etienne, Alain**
**24 Route de Pegomas**
**F-06130 Grasse (FR)**

(74) Representative : **Bullows, Michael**
**Dow Corning Limited Cardiff Road**
**Barry South Glamorgan CF6 7YL Wales (GB)**

## Description

This invention is concerned with sustained release elements and their manufacture.

Numerous proposals have been made for sustained release products especially, for example, in the field of delivery of therapeutic and/ or diagnostic materials to the human or animal body. Prior proposals include for example, external application of a preformed transdermal patch to the body, insertion of a preformed implant into the body, attachment of a dressing to the tissue of a cavity and oral administration of a preformed element containing an active substance intended for administration orally.

In the field of controlled release of therapeutic and diagnostic agents into the human or animal body from implants, it is known to employ silicone based materials as a matrix or barrier through which a lipophilic agent is able to diffuse at a controlled rate into the body. Silicone based materials proposed for the purpose are generally inert to body fluids and therefore highly acceptable for use in the body. However, the rates at which therapeutic and other compounds are released from or through silicone materials are generally very low due to the diffusion characteristics from a silicone matrix and are generally considered to be "first order" i.e. the diffusion rate is a function of the square root of time. This is beneficial for controlled release of those therapeutic and other agents which are required to be introduced to the body at a comparatively low rate over a long period of time. However, it would be beneficial if a wide range of therapeutic and other agents could be delivered into the body at a comparatively high rate of more than several mg to 1g per day for one or more days or even weeks, at an at least substantially constant rate independent of time, which is to say at zero order release rate. For these requirements, the silicone based materials employed hitherto have been regarded as unsuitable due to the need to employ lipophilic agents whose rate of diffusion through the silicone material is very limited.

E.P. 231 286 discloses a pharmaceutical delivery device in the form of an elastomeric silicone matrix capable of dispensing hydrophobic or hydrophilic active agents when certain surfactant materials are employed during its manufacture. The devices are able to accept higher loadings of active agent, a factor which effects the subsequent release of active agent. It has been found that such a device is capable of releasing an active agent over a prolonged period of time consistent with the higher loading of active agent.

E.P. 277 740 discloses an aqueous coating composition for forming an elastomeric coating around a core of active agent (e.g. drugs) comprising a dispersion of polyorganosiloxane latex particle, colloidal silicon particles and a water dispersible organic material. The elastomeric coating is used to control the release rates of an active agent into a surrounding aqueous medium. Such a device, it has been recited, is capable of delivering drugs with a substantially zero order release rate.

The administration of drugs to the human or animal body by way of sustained release from a carrier located in the gastrointestinal tract of the human or animal body has long been an objective. It is generally acknowledged that residence time of a drug in the stomach is largely unpredictable as it depends on the physiology of the individual and upon the amount and type of food which is taken with a meal. The natural housekeeping motions of the stomach tend to expel matter from the stomach every second hour. For those drugs which are also effective in the small intestine, the maximum period during which the drug is liberated is extended by up to about 4 hours, after which the drug passes to the colon where the pH conditions are markedly different. Accordingly, it would be advantageous if one were able to provide means for sustained, controlled release of a drug in the stomach over many hours irrespective of the physiological conditions in the stomach at the time of administering the drug preparation, and to defer its expulsion, and thus emission of unconsumed drug, from the stomach for an extended period of time. Numerous proposals have been made with a view to reaching this objective, but we are not aware of any universal commercially satisfactory solution especially in respect of delivery of drugs which are only slightly soluble at acidic pH or which can be absorbed only when the surrounding pH is greater than 5.

It has been proposed to form tablets for consumption by humans or animals by compression of quantities of capsules or particles of medicament together with excipient with a view to achieving satisfactory administration of required doses of active material. However, many film forming materials proposed for use in production of capsules and coated particles are somewhat brittle and incapable of withstanding the compressive forces of processes employed for making tablets, so that formation of tablets from capsules or particles intended to have controlled release characteristics may result in release characteristics less than desired due to presence in the tablets of at least some damaged capsules or particles.

It is one of the objects of the present invention to provide improved sustained release elements for controlled release of agents in aqueous media, especially for example, therapeutic and diagnostic agents.

It is another object of the present invention to provide improved means for sustained release of therapeutic and diagnostic agents in aqueous media and particularly in aqueous media exemplified by the biological fluids found in the stomach or gastrointestinal tract of man and or animals.

It is another object of the present invention to provide improved means for sustained release of therapeutic and diagnostic agents to the human or animal body at a location where copious quantities of aqueous media are not normally present, for example body cavities and mucosa.

It is another object of the present invention to provide improved sustained release elements in the form of particulate material capable of being suspended in the stomach, or in a particulate or other form suitable for insertion in the human or animal body as an implant or as an insert in a body cavity.

We have now found that a vehicle formed from a silicone polymer a hydrophylic component and a modulating component offer enhanced opportunities for controlled release of active substances in the body.

The present invention provides in one of its aspects a sustained release element comprising an agent (A) capable of release from the element when the element is in an aqueous medium, a cured silicone material and a hydrophilic organic material characterised in that the element consists of a vehicle (B) comprising a resilient silicone polymer body having mixed therein the agent (A), the polymer body being formed from a curable composition comprising a polysiloxane containing alkyl hydrogen siloxane units, a polysiloxane containing unsaturated groups for reaction therewith, and a platinum or rhodium catalyst, 5 to 40% by weight of the vehicle of a hydrophilic component and up to 40% by weight of the vehicle of a modulating component which serves to modulate the release of agent (A) selected from (I) an organic hydrophilic substance having two or more hydroxyl groups per molecule and/or (II) an organic hydrophilic polymer which swells in an aqueous medium.

Sustained release elements according to the invention comprise an agent (A) capable of release from the element when the element is in an aqueous environment. Elements according to the invention are particularly useful for those agents (A) which are not soluble in or do not diffuse sufficiently through silicone materials i.e. those materials which are hydrophilic. Whilst the invention may be employed in the sustained release of a variety of materials, for example perfumes, pesticides, bacteria, yeasts, and processing aids in a variety of trades and industries, it is particularly beneficial for delivery of therapeutic, diagnostic or nutritive agents and the like to living organisms, particularly mammals animals, fish and cell cultures. The following description of sustained release elements for use in therapeutic or diagnostic methods for the human or animal body, wherein the agent is a therapeutic agent and the aqueous medium referred to is a biological fluid normally present in the body which may have a pH from about 1 to about 7 and may contain various ionic and other materials, is given by way of example to illustrate particular application of the invention to uses in the human or animal body.

Elements according to the present invention intended for delivering therapeutic or diagnostic agents to the human or animal body may have any convenient form suitable for administration to the body. For administration to the gastrointestinal tract, an element may take the form of a capsule or tablet or other three dimensional form suitable for oral administration or it may take the form of finely divided particles or microcapsules, which may be incorporated in tablets or capsules, for example capsules of gelatin of the type and size generally employed in pharmacy for oral administration. For external application e.g. for transdermal use or for fitting in an artificial or natural body cavity, for example the occular, buccal, nasal, aural, vaginal or rectal cavity, the element may take the form of a preformed pad, patch, thread, film, tape, ring or matrix or it may take the form of finely divided particles or microcapsules. Elements according to the invention may be shaped as by moulding, extrusion, pelletising or granulating or in any other convenient way. The element may be made by injection moulding, by casting the composition into a mould or by extrusion followed by shaping or cutting the extrudate into required shapes using a thermoplastic or curable formulation. Particles, e.g. of 1mm or less, may be formed by wet granulation or coating pan granulation techniques in which the components of the vehicle are masticated between blades, by ejection of a mixed formulation into a fluidised bed as the composition sets or cures or by emulsion polymerisation techniques. In order to facilitate preparation of particles by granulation, one may include in the formulation from which the particles are prepared a volatile or non volatile process aid, for example a silicone fluid eg a trimethylsilyl end-blocked polydimethyl siloxane, or an organic liquid. Volatile process aids are preferred. The particles may be used to make capsules, tablets or other dosage forms, e.g. suspensions, ointments or suppositories, by conventional techniques. Characteristics of the elements may be controlled within wide limits by appropriate selection of the components and the method of manufacture.

The silicone polymer of the vehicle of an element according to the invention serves as a binder matrix for the other components and ingredients and may be employed in proportions to generate a greater or lesser proportion of the binder matrix depending on the intended use of the element. When producing elements by coating or granulation techniques, it is generally not possible to incorporate more than about 25% of liquid components with the solids employed in a single coating or granulating step, whereas in those cases where moulding type techniques are employed in which the solid ingredients are dispersed in liquid ingredients prior to moulding, one may employ larger proportions of the silicone. For articles in pellet, powder or similar finely divided form which are intended for incorporation into tablets by a compression process, the matrix should be sufficiently elastomeric to preserve the integrity of the finely divided form without bursting, whereas for some other forms it is important that the matrix contribute to shape retention of the element, whereas in yet others it may be important that the matrix have truly elastomeric properties. In those cases where the element is intended for use in the gastrointestinal tract, one need employ the silicone polymer in no more than minor amounts, for example 5% to 50% by weight of the vehicle whereas for elements formed to fit into a body cavity or as an implant one may employ more of the silicone polymer, for example 40 to 95% by weight of the vehicle, to ensure sufficient shape retention during use or to restrict the rate of release of the agent (A).

Elements according to the invention are made from a formulation comprising the components of the vehicle (B) in admixture with the agent (A). If desired the formulation may comprise additional ingredients for example fillers (which may be, for example opaque to X rays or other diagnostic radiation) and excipients employed in pharmacy and compounds intended to perform as pH buffers in controlling the environment immediately in and around the element when it is in an aqueous environment. The formulation may comprise a silicone composition which cures to provide the silicone polymer and may be designed to cure within a desired, for example short, period of time at a desired operating temperature e.g. in the range 15°C to 110°C. The components and other ingredients and the proportions thereof are preferably selected so that the silicone polymer is sufficiently developed and cured to a desired form retaining structure within a short period of time of the order of ten minutes or less.

The agent (A) may be a solid or liquid material and may be combined with the vehicle after preparation of the vehicle, for example by aspiration into a cellular element, but is preferably incorporated into the vehicle before or during preparation of the vehicle. In a preferred mode of preparing the elements, the agent (A) is introduced to the components of a curable silicone based formulation for the vehicle prior to or during curing thereof. If it is intended to prepare elements by curing the silicone polymer in presence of the agent (A), it is important to ensure that the agent (A) chosen does not interfere with the curing of the silicone to an unacceptable extent. The invention is especially applicable to those therapeutic and diagnostic agents which it is desired to deliver to the body over a period of time at a controlled rate. As is known, the rate of delivery required of a given drug falls within a therapeutic window. By tailoring the formulation of the vehicle of an element according to the invention, it is possible to provide elements from which many drugs can be delivered at rates within their therapeutic window. Therapeutic or diagnostic agents suitable for use as the agent (A) of the present invention include those which are intended to be released into the body via the blood stream or from the gastrointestinal tract and may be hydrophilic or lipophylic materials. The agent (A) may be chosen in accordance with normal pharmaceutical practice and will normally have a pH appropriate to the conditions at the region in the body where it is to be released. If appropriate, the pH of the substance may be buffered in order to preserve its activity. Therapeutic agents which may be employed include for example antibiotic, antiseptic, antiinflammatory, cardiovascular, antihydrogen, bronchodilator, analgesic, antiarrythmic, antihistamine, $\alpha$-1 blocker, beta blocker, ACE inhibitor, diuretic, antiaggregant, sedative, tranquiliser, anticonvulsant and anticoagulant agents, vitamins, agents for treating gastric and duodenal ulcers, proteolytic enzymes, healing factors, and peptides. Specific examples of suitable therapeutic agents include penicillins, cephalosporins, tetracyclines, macrolides, epinephrine, amphetamines, aspirin, barbiturates, catecholamines, benzodiazepine, thiopental, codeine, morphine, procaine, lidocaine, sulphonamides, tioconazole, perbuterol, furosamide, prazosin, prostaglandins, salbutamol, indomethicane, diclofenac, glafenine, dipyridamole and theophylline. In other applications the agent (A) employed may be an agent appropriate to the intended use of the elements, for example an agent essential to life support or nutrition of organisms present in the liquid medium.

Some of the operative therapeutic and diagnostic agents may contribute to the activities of the hydrophylic or modulating components and may provide, for example, the modulating component.

The proportion of the agent (A) employed in an element according to the invention is chosen in accordance with the concentration of the agent (A) required to deliver the dosage required at the proposed delivery rate and may be varied within a very wide range. The agent (A) may provide a major or a minor amount of the element. The efficient delivery achieved with elements according to the invention permits use of comparatively low dosage levels. However, it is generally desirable to include as large a proportion of therapeutic or diagnostic agent as possible consistent with the desired delivery of the agent.

In an element according to the invention, release of the agent (A) is dependent upon presence of water in the environment in which the element is present. When the elements are stored in an anhydrous environment, no swelling occurs and at least substantially no release of the agent (A) takes place, whereas when located in an aqueous environment the element swells, at least to a small extent, as a result of intake of water and then the agent (A) is progressively released. The rate at which the agent (A) is released appears to be dependent upon the surface area of the element and the extent to which the element swells (and swelling is dependent upon the quantity and pH of the aqueous environment of the element), and upon the dissolution rate and/or the contribution to hydrophilic or modulating activity of the agent (A), upon the nature of the hydrophilic component and upon the nature of the modulating component. The mechanism by which this phenomenon occurs is not fully understood. However, without wishing to be bound by any particular theory, we believe that in one aspect, the resilient silicone polymer and the hydrophilic component in combination are essential to satisfactory sustained release through a combination of elastomeric and osmotic properties, the hydrophilic component serving to draw water into the element, for example to provide a hypertonic environment, and to cause the silicone polymer to become mechanically distended, and the force of contraction of the distended silicone polymer serves to deliver the agent (A) to the surrounding medium from the element.

The modulating component serves to modulate the expulsion of the agent (A) and may be selected in accordance with the modulation desired. For example, those materials which swell substantially in water may be expected to modulate the delivery of the agent (A) to enhance the period during which the agent is delivered at a somewhat high rate, with a consequently short overall useful life of the element, whereas those materials which do not swell substantially in water may be expected to modulate the delivery of the agent (A) to improve the constancy of the delivery of the agent (A) and the proportion released and possibly also lengthen the overall useful life of the element.

In an element according to the invention, the hydrophilic component is preferably a liquid at relevant temperatures, but solid materials (for example sorbitol, manitol, sodium chloride and certain drugs) dissolved in suitable solvent may be used. The hydrophilic component is preferably an organic hydrophilic material having two, more preferably three or more hydroxyl groups per molecule and may be chosen, for example, from the liquid polyethylene glycols having a molecular weight in the range of 100 to 600, propylene glycol, glycerol, sorbitol and manitol. The hydrophylic component is preferably a hygroscopic material. Whilst some swelling and release of the agent (A) takes place from elements having one of these as the hydrophilic component, we prefer to use a material which is a liquid at the temperature at which the agent is to be released, and preferably also liquid at the temperature at which the element is made, and to use a material which permits use of curable silicone compositions to make the vehicle (B). This component is most preferably selected from the water soluble, polyhydric alcohols having a melting point of less than 25°C. The most preferred material is glycerol. In order to ensure release of the agent (A) at a desired rate when the elements are exposed to an aqueous environment, the hydrophylic component is employed in the formulation employed to prepare the vehicle (B) in a proportion within the range of 5 to 40% by weight of the vehicle. If less than 5% by weight of the hydrophilic component is present, the beneficial effects are not realised, whereas if more than 40% is employed not only are the beneficial effects not realised, but also the cure characteristics of a curable silicone formulation (when used to make the element) may be very adversely influenced and/or some or all of the hydrophilic material may be lost from the element. Proportions within the range specified may be selected with regard to the intended life cycle time of the elements. In general, when glycerol is used, we prefer to employ from 10% to 25% by weight glycerol in the formulation employed to prepare the vehicle (B).

The modulating component may be selected from a wide range of organic materials. Modulating components which serve to dampen the initial burst of agent (A) from the element include organic hydrophilic substances (I) having two or more hydroxyl groups per molecule. Modulating components which serve to promote release of the agent (A) at a high rate include hydrophilic polymers (II) which swell in an aqueous medium. These substances (I) and polymers (II) serve to modify the release characteristics of the vehicle and to modulate the release in different ways. The particular substance (I) or polymer (II) used and the proportion employed in the formulation used to prepare the vehicles are selected in accordance with the rate of delivery and the period during which delivery is required. In an element according to the invention, the substance (I) serves to regulate or eliminate the initial burst of the agent from the element when introduced to an aqueous medium and to extend the life of the element during which the drug is delivered at a constant and optionally comparatively high rate. In contrast, the polymer (II) serves to increase the swelling of the element when the element is introduced to aqueous media of selected pH and consequently serves to increase the ability of the elements to discharge the agent at a very high rate in media of that pH. Accordingly, for those elements which are intended for use in delivering agents (A) at a rate of the order of 1 to 10 mg per day for 7 days to 10 days or more, and for those elements intended for use in environments where copious quantities of aqueous media are not normally present, for example natural cavities in the body, we prefer to employ a substance (I). Conversely, for those elements which are intended for use in delivering agents (A) at a rate of the order of 10 to 1000 mg per 24 hours, and for those elements intended for use in the gastrointestinal tract, where copious quantities of aqueous media are normally present, we prefer to employ a polymer (II). The modulating component may be selected in accordance with the pH of the aqueous medium in which the agent (A) is to be delivered. It is known that regions of the human or animal body normally have specific pH characteristics. For example, in the gastrointestinal tract the fluids in the stomach usually exhibit a pH of 1 to about 2 when the stomach is fasted and about 3 to about 5 when the stomach has been fed. The fluids in the small intestine usually exhibit a pH in the range of about 4 to about 6.5, those in the large intestine a pH of about 7.0 to 7.5. In contrast, the aqueous media which are available in comparatively small volumes in cavities of the body tend to have more neutral pH values of the order of about 6 to about 7.5.

The organic hydrophilic substance (I) may have two, more preferably three or more hydroxyl groups per molecule and may be chosen, for example, from the polyethylene glycols having a molecular weight in excess of 1500, sorbitol, manitol, lactose and mixtures thereof. The material used is selected in accordance with the characteristics required of the element and especially the release profile desired in the selected location for the element. Whilst each of these materials modifies the delivery of the agent (A) from elements according to the invention, we prefer to use a material which is a solid at the temperature at which the vehicle is prepared and the temperature at which the agent (A) is to be released. We also prefer to employ a material which is hygroscopic. The material is most preferably selected from the water soluble, polyhydric alcohols having a melting point of greater than about 40°C. The most preferred material (I) is sorbitol. In order to ensure release of the agent (A) at a desired rate when the elements formed therefrom are subjected to an aqueous environment, we prefer to employ the sorbitol in the formulation from which the vehicle is made in a proportion of up to 40%, more preferably from 5 to 25% by weight of the formulation.

The hydrophilic polymer (II) may be any one or a mixture of any two or more of those organic polymers known to be capable of swelling in aqueous media provided that it does not interfere with preparation of the vehicles, for example when a curable silicone composition is employed, and provided that an adequate degree of swelling is achieved in a medium having a selected pH. Generally, in order to achieve prolonged fast delivery of the agent (A) it is preferred that the polymer exhibit significant swelling as determined by its water imbibition during a short time. For example, for release of agent (A) in the stomach, elements capable of swelling to double their swelling index within two hours are extremely useful. The polymer may be chosen, for example, from the group consisting of cellulosic materials, e.g. cellulose and cellulose derivatives for example carboxy methyl cellulose, sodium carboxymethyl cellulose whether crosslinked or not, hydroxypropylcellulose and acetlyated chitin. Some of these materials swell irrespective of the pH value of the medium in which they are placed, but the degree of swelling exhibited by others, for example ionic salts, is dependent on the pH of the aqueous medium. Additionally, the rates at which these various materials swell upon insertion in aqueous media differ one from another. The swelling characteristics of the different available materials may be beneficially selected so that a desired degree of swelling may be achieved in a region of the gastrointestinal tract within which the aqueous medium has a certain pH value. In one aspect, the element may thus be caused to preserve a mode where the rate of release is very slow until it becomes located in a region of predetermined pH value whereupon it swells at least to a limited extent and the agent (A) is released at a desired higher rate. In another aspect, the element may be made to swell within the stomach to assume a size sufficient to prevent its expulsion from the stomach by the natural peristaltic movements so that the agent(A) may be released at a controlled high rate into the gastric juice in the stomach for absorption by the stomach or subsequent regions of the gastrointestinal tract. These elements can be retained in the stomach for some hours or days and during this time the element becomes flexible and spongy enough so that delivery of the agent (A) from the element in the stomach may continue over a comparatively long period, for example 12 to 24 hours. We prefer to employ sodium carboxymethyl cellulose for elements intended for delivery of drugs within the gastrointestinal tract, and to use it in proportions of 10 to 25% by weight of the vehicle. Sodium carboxymethylcellulose does not swell significantly in strongly acidic medium, but at pH of about 4 to 9 it swells to two or three times its unswollen v olume. Using this material we have found that one may achieve preferential release of a therapeutic or diagnostic agent at pH 4 or greater with low release at very acidic pH and with zero order release at both these pH values.

Modification of the physiological pH at which the element swells, for example in the gastrointestinal tract may be effected by inclusion in the element of a salt which serves as a buffer. The substance chosen is selected from those which do not unduly affect the process by which the element is prepared and which do not adversely influence desired properties of the elements. We have found that by incorporating sodium acetate in the formulation, swelling of elements comprising sodium carboxymethylcellulose as modulating component may be induced to a desired extent to release agent (A) in aqueous medium of pH 4 or less (such as may be encountered in the stomach).

Certain of the swellable polymers (II) suitable for use in the invention, for example, certain of the cellulosic materials, contribute not only to swelling and to the ability of the element to release the agent (A) but also to bioadhesive characteristics of the drug delivery element, i.e. the ability of the vehicle to adhere to mucosa in body cavities. Bioadhesive characteristics of the element may also be promoted by presence in the vehicle of certain selected polymers and copolymers of acrylic acid (e.g. polyacrylic acid cross linked with polyalkyl sucrose or 3,4-dihydroxy-1,5-hexadiene), acrylates e.g. poly(hydroxyethyl methacrylate), vinylpyrollidones, vinyl acetate, polycarboxylic acids, poly(ethylene oxide), alginates, gelatin, pectin, pectin derivatives, natural gums, proteins, pharmaceutically active salts of these and mixtures thereof. The property of greater or lesser adhesion of the swollen element to the body is pertinent to the intended mode of use of the element. For example, in some cases it may be advantageous to have good adhesive properties to ensure that the element adheres well to external or internal body tissues, whereas in other cases it may be advantageous if the adhesive properties are poor, for example where the element is intended to pass through a portion of the gastrointestinal tract prior to arrival at the intended site of maximum drug release. The adhesive properties conferred also appear to be dependent on the degree of swelling and therefore pH dependent. For example, elements according to the invention which contain sodium carboxy methylcellulose as modulating component are substantially non adhesive to body tissues in aqueous medium of pH 1 to 3.5, but adhere well in aqueous media at pH 4 to 8. In addition to the possibility to design the elements for bioadhesion in selected portions of the gastrointestinal tract, e.g. for colonic delivery, it will be apparent to those skilled in the pharmaceutical art that elements intended for external application to the body which contain a hydrophilic substance (I) as modulating agent and which are not intended to swell substantially, may also contain a swellable polymer (II) to induce bioadhesion. This may be especially beneficial, for example, in particulate elements intended for application to the buccal, nasal or occular cavities and possibly to the rectal or vaginal cavities where the extent of swelling is restricted by the smaller proportions of aqueous medium present, or in the gastrointestinal tract.

Silicone polymers present in a sustained release element according to the invention are those suitable for binding, i.e. containing or entrapping, the other components of the composition, and may provide a major or minor proportion of the vehicle. The silicone polymer is somewhat resilient and may be a thermoplastic copolymeric material or a homo- or copolymeric material in the form of a gum, resin or elastomer. Preferred silicone polymers are resinous or elastomeric materials formed from silicone compositions which may be cured to cellular or non-cellular form when in admixture with the other ingredients employed to prepare the vehicle or the sustained release element. Those compositions which can be cured at temperatures no greater than 100°C are preferred, as degradation by strong heating of heat sensitive ingredients present in the curing mass, for example drugs, may thus be avoided. For example, those silicone compositions which are curable at room or slightly elevated temperatures eg by way of curing of a one or two part composition or by way of irradiation techniques, are appropriate when production of the elements without heating is desired. An especially facile production may be achieved when the formulation from which the vehicle is made is formulated to cure at room temperature within a few minutes of mixing. Those silicone compositions used are curable without use of a tin compound as catalyst and employ polysiloxanes having silicon-bonded unsaturated organic groups, e.g. vinyl groups, available for reaction with polysiloxanes having silicon bonded hydrogen atoms in presence of a hydrosilylation catalyst, for example a platinum or rhodium compound. The addition reaction which occurs is appropriate to yield chain extended or cross linked unfoamed resinous or elastomeric silicone products.

Suitable polydiorganosiloxanes having unsaturated groups for reaction with polydiorganosiloxanes having silicon-bonded hydrogen atoms include polydiorganosiloxanes which have sufficient unsaturated groups for formation of the polymer network, for example polysiloxanes having siloxane units according to the general formula $R_mR'SiO_{\frac{(3-m)}{2}}$ in which each R represents a monovalent hydrocarbon group having up to 20 carbon atoms, for example a lower alkyl, e.g. a methyl group or phenyl group, $\underline{m}$ is 1 or 2 and R' represents an aliphatically unsaturated group, for example cyclohexenyl or a group R"CH=CHR''', where R" represents a divalent aliphatic chain linked to the silicon atom and R''' represents a hydrogen atom or an alkyl group for example vinyl, allyl, or hexenyl. The group R' may thus be for example, vinyl, allyl or hexenyl. These polysiloxanes also comprise units $R_nSiO_{\frac{(4-n)}{2}}$ in which R is as referred to above, and $\underline{n}$ is 1, 2 or 3. Preferably, these polysiloxanes have from 0.01% to 1% by weight of aliphatically unsaturated groups and a viscosity of the order of about 10 $mm^2/s$ to about 25,000 $mm^2/s$. More preferably their viscosity lies in the range 100 $mm^2/s$ to 2,000 $mm^2/s$.

Suitable polydiorganosiloxanes having alkylhydrogen-siloxane units include polymers having units according to the general formula $R_pHSiO_{\frac{(3-p)}{2}}$ in which each R represents a monovalent hydrocarbon group containing 1 to 20 carbon atoms, for example a lower alkyl e.g. a methyl group or phenyl group and $\underline{p}$ is 1 or 2. The alkylhydrogen polysiloxanes may also comprise units $R_nSiO_{\frac{(4-n)}{2}}$ as referred to above.

Preferably this polysiloxane has from 0.5% to 2.5% by weight of silicon-bonded hydrogen atoms. We prefer that each R represents a methyl group. Preferably, terminal groups of the alkylhydrogen polysiloxane have the formula $R_3SiO_{\frac{1}{2}}$ where each R represents a methyl group. Suitable alkylhydrogen polysiloxanes include those comprising MeHSiO units with or without the presence of $Me_2SiO$ units and having viscosities of the order of from about 1 to about 1000 $mm^2/s$ more preferably from about 5 to about 50 $mm^2/s$.

In order to achieve satisfactory cure, it is important that the ratio of silicon bonded hydrogen atoms of the polysiloxanes to all groups reactive therewith in the composition is appropriate, so that enough of the alkylhydrogen polysiloxane is present to effect the desired cure. We have found that formulations for preparing the elements may be formulated to cure within three minutes or less of mixing of the composition at room temperature (i.e. of the order of 22°C) and humidity (i.e. about 60% to 80% relative humidity), although the curing time is dependent on various factors, including the type and proportion of other components present in the curing mixture and especially the salt materials which tend to retard the cure significantly. The rate at which the agent (A) is released is also dependent to some extent upon the resilience of the silicone polymer.

Platinum catalysts may take any of the known forms, ranging from platinum as deposited on carriers such as silica gel or powdered charcoal, to platinic chloride, salts of platinum and chloroplatinic acids. A preferred form of platinum is chloroplatinic acid either as the commonly obtainable hexahydrate or the anhydrous form, on account of its easy dispersibility in organosilicon systems and its non-effect on colour of the mixture. Platinum complexes may also be used e.g. those prepared from chloroplatinic acid hexahydrate and divinyl tetramethyldisiloxane. If it is desired to prolong the cure time one may include in the composition one of the known platinum catalyst inhibitors such as a polymethylvinylsiloxane cyclic compound or an acetylenic alcohol eg methyl butynol. The proportion of catalyst and inhibitor used in the composition influences the rate of curing of the silicone formulation and also influences the consistency and elastomeric properties of the cured formulation. The rate of cure of the formulation from which the vehicle (B) is formed is dependent not only upon the silicone polymer forming ingredients but also upon the nature of the other ingredients of the mixture. In particular the nature of the agent (A) which is to be released, and of any salts present during the curing. Presence of ionic salts, e.g. sodium acetate, in formulations for providing the vehicles tends to extend the cure time and in such cases it is desirable to reduce or eliminate the proportion of inhibitor present and/or to increase the proportion of platinum catalyst employed.

In those cases where it is desired that the element have a low density, for example when release of the agent (A) from the element is required to occur whilst the element is suspended or floating in an aqueous medium, for example in the gastric fluid in the stomach, it may be desirable to ensure that the cured silicone formulation is cellular and contains closed cells. Foaming may be induced by inclusion among the silicone forming materials of a polydiorganosiloxane having silicon-bonded hydroxyl groups with a view to reaction with the polydiorganosiloxane having silicon-bonded hydrogen atoms as more fully described for example in U.S. 4 026 845, and/or by inclusion of water or an aliphatic alcohol (for example, a primary aliphatic or araliphatic alcohol for example a lower aliphatic monofunctional alcohol having up to 12 carbon atoms, e.g. ethanol, n-propanol, or benzyl alcohol) or by inclusion in the composition of a volatile blowing agent as more fully described for example in U.S. 4,550,125. Preferred foamable formulations include compounds having silicon bonded or carbon bonded hydroxyl groups which foam and cure in presence of a platinum catalyst according to the scheme $\equiv$SiH + HOQ ----> $\equiv$SiOQ + H$_2$. The group Q may be for example an aliphatic group or a polysiloxane having one or more reactive hydroxyl groups so that by virtue of the plurality of silicon bonded or carbon-bonded hydroxyl groups the hydrogen evolved as a gas serves to form cells within a developing network of interconnected polysiloxane chains. Curable silicone formulations employed for preparation of cellular elements may also comprise foam stabilisers or surfactants. Suitable materials include fluorinated silicones. The cellular formulations may be formed into particles, for example, by granulation techniques or by emulsion polymerisation in water in presence of a surfactant.

If desired other adjuvants may be incorporated in the silicone compositions for example fillers, colorants, coloured indicators, inert extenders, diluents and processing aids for example cyclic and linear polydiorganosiloxanes. The presence of some silica filler is desirable when elements of strongly elastomeric properties are required.

Preferred formulations for use in preparing elements according to the invention are curable at room temperature when mixed, and therefore are normally used to produce sustained release elements immediately upon mixing the various components. In those cases in which it is desired to store the formulation prior to admixture and formation of a drug delivery element, this may be achieved by storing the formulation in separate parts one of which contains the catalyst for the curable silicone composition and one of which contains the alkylhydrogen polysiloxane. When the agent (A) is present in the formulation, it may be preferable to include it in one only of the parts of the formulation in order to preserve its effectiveness.

An element according to the invention in a form suitable for oral administration preferably comprises the components (A) and (B) in a proportion of from 0.5 to 95, more preferably up to 80, parts by weight of the agent (A) to 99.50 to 5 parts by weight of the vehicle (B), the vehicle (B) comprising 10 to 35% by weight of the hydrophilic component, 10 to 60% by weight of the silicone polymer and 10 to 40% by weight of a modulating component selected from hydroxypropyl cellulose, carboxymethyl cellulose or sodium carboxy methyl cellulose whether crosslinked or not and mixtures thereof.

An element according to the invention in a form suitable for administration to a cavity of the body preferably comprises the components (A) and (B) in a proportion of from 0.5 to 80, more preferably up to 60 parts, by weight of the agent (A) to 99.5 to 40 parts by weight of the vehicle (B), the vehicle (B) comprising 10 to 40% by weight of the hydrophilic component, 40 to 80% by weight of the silicone polymer and 10 to 40% by weight of a modulating component selected from polyethylene glycols of molecular weight greater than 1500, sorbitol, manitol and lactose and mixtures thereof.

The present invention offers numerous advantages. The silicone and other materials chosen enable simple and easily controlled methods of manufacturing sustained release elements at room or elevated temperatures to a chosen density, size and shape and having selected combinations of properties (e.g. flotation, bioadhesion, release rate and release profile) without imposing severe processing conditions upon incorporation into the vehicle of the substance to be released, e.g. high temperatures or pressures, which might be damaging to medicaments used. The silicone materials and other ingredients used are acceptable in the human or animal body. The elements may be formulated to give a moderate to rapid release of agent (A), which is in many cases highly advantageous in that high zero order release rates of from 1 to 10 mg per day over 7 to 30 days or of from 10 to 1000mg per day may be achieved. The release may be caused to occur in selected regions of the body, giving rise to the opportunity for targeted delivery, e.g. in the stomach, colon or mucosa, of therapeutic or diagnostic agents. The drug delivery profile of elements according to the invention may be predetermined by appropriate selection of the types and proportions of components and ingredients used. A particular advantage of elements according to the invention is their ability to release agent (A) at a controlled rate substantially better than heretofore achieved with silicone materials. We have found for example that both lyophilic and hydrophilic agents may be released from the elements at a delivery rate of the order of 100 mg or more per day. It is a further advantage of the present invention that the elements can be elastomeric particulate materials able to withstand pressures exerted during the tabletting process. It is a further practical advantage that the elements may be formulated to be retained within the stomach for prolonged periods, thus giving enhanced opportunity for prolonged sustained release of therapeutic or other agents into the stomach. With regard to elements for use in the gastrointestinal tract, it is possible to provide dosage forms of a size and physical properties suited for easy oral administration the element or elements of which become enlarged and retained in the stomach, and which break down slowly in the gastrointestinal tract by erosion of their organic components to leave a flexible remnant of silicone which is easily discharged from the body.

In order that the invention may become more clear there now follows a description of various formulations and compositions, Examples 8, 9 and 10 being illustrative of the invention. In the Examples all parts and percentages are expressed by weight. The Examples show the dependence of swelling and density upon the components of elements made by various techniques from formulations containing silicone compositions and upon the pH of the medium in which the elements are placed. The Examples also show dependence of drug delivery upon swelling.

The silicone materials employed in the Examples were as follows:

Silicone Part 1A comprised 69 parts of dimethyl vinyl endblocked polydimethylsiloxane fluid having a viscosity at 25°C of about 2,100 mm$^2$/s, 6 parts hexamethyl disilazane, 1 part water, 0.1 part of a platinum catalyst, being a complex of chloroplatinic acid hexahydrate and vinyl siloxanes, and 24 parts of fume silica;

Silicone Part 1B comprised 88 parts of the dimethylvinyl endblocked polysiloxane, 12 parts of a copolymer of polydimethyl and polymethylhydrogen siloxanes having a viscosity at 25°C of about 5 mm$^2$/s, 0.75% hydrogensiloxane units (as % H) and 0.4 part methylvinyl cyclic polysiloxanes;

Silicone Part 2A comprised silicone Part 1A with an additional 1% of the platinum catalyst;

Silicone Part 2B comprised silicone Part 1B without the cyclic siloxanes;

Silicone Part M1A comprised 46.7 parts of a dimethylvinyl endblocked polydimethylsiloxane having a viscosity at 25°C of 450 mm$^2$/s, 1.4 parts ethyl alcohol and 1.9 parts of the platinum catalyst;

Silicone Part M1B comprised 32.25 parts of the vinyl polysiloxane used in Part M1A, 8 parts of a trimethyl silyl endblocked methylhydrogen polysiloxane having a viscosity of about 40 mm$^2$/s, 8 parts of the copolymer of polydimethyl and polymethylhydrogen siloxanes used in Part 1B, 1.75 parts of a surfactant being the reaction product of a resinous dimethylsiloxane copolymer and an alcohol having the formula $F(CF_2)_8CH_2CH_2OH$ as more fully described in E.P. 179 598 and 40x10$^{-3}$ parts methylbutynol.

Cellulosic materials used in the Examples were as follows:

Cellulosic material 1 was a sodium carboxy methyl cellulose supplied under the trade name Tylopur 1000 by Hoechst;

Cellulosic material 2 was a hydroxypropylmethyl cellulose phthalate supplied under the trade name HP50 by Seppic having an average molecular weight of 20,000 and a carboxybenzoyl groups content of 20 to 24%;

Cellulosic material 3 was a hydroxypropylmethyl cellulose phthalate supplied under the trade name HP55 by Seppic having an average molecular weight of 20,000 and a carboxybenzoyl groups content of 27 to 35%;

Cellulosic material 4 was a blend of microcrystalline cellulose and sodium carboxymethyl cellulose supplied under the trade name Avicel RC591 by Seppic having an average particle size of 28 micrometers;

Cellulosic material 5 was a crosslinked sodium carboxy methyl cellulose supplied under the trade name ACDI SOL by Seppic having a degree of substitution of 0.6 to 0.85;

Cellulosic material 6 was a hydroxypropylmethyl cellulose supplied under the trade name METOLOSE 60S H50 by Seppic having an average molecular weight of about 8,600; and

Cellulosic material 7 was a methyl cellulose supplied under the trade name METOLOSE SM15 by Seppic having approximately 30% of methoxy groups.

Discs were made from formulations for swelling, bioadhesion and release studies. The discs were prepared by mixing the components of each formulation, for example using an Heidolph RGL 500 bench homogenizer. The mixed formulations were pressed into a mould between two 125 micron polyester films then cured to give a matrix with a thickness 2 to 2.2mm and 2cm discs were punched therefrom. The discs were cured at 100°C for 10 minutes unless otherwise stated. To determine swelling, unless otherwise stated, each disc was weighed then placed into 200ml of de-ionised water at 20°C or other aqueous medium, being a buffered solution. Each disc was removed, all water removed from its surface using a filter paper and then weighed again. The Weight Swelling Ratio Ws/Wd is recorded as the ratio between weight in the swollen (Ws) and dry (Wd) state.

Solutions referred to as having specified pH were prepared as follows:

pH = 1.2 and pH = 2 were made from a KCl, HCl buffer (Carlo Erba);

pH = 3, 4, 5 and 6 were made from a potassium biphthalate buffer (Carlo Erba);

pH = 7 and 8 were made from $KH_2PO_4$ buffer (Carlo Erba);

pH = 4.5 was made according to USP XXI from an acetate buffer.

Example 1

The swelling and bioadhesive properties were examined of discs made from formulations comprising 60 parts of a mixture of silicone Part 1A and 1B in a ratio of 3:1.5, 25 parts of cellulosic materials 1 to 7 and 15 parts of glycerol. The discs were allowed to swell in de-ionised water at 22°C for 48 hours. Results are shown in Table 1.

## TABLE 1

| Cellulosic Material | Ws/Wd | W% | Remarks |
|---|---|---|---|
| 1 | 6.3 | 530 | Slight tack gel like surface |
| 2 | 6.4 | 540 | Dry surface |
| 3 | 3.5 | 250 | Dry surface |
| 4 | 4.6 | 360 | Dry surface |
| 5 | 6.6 | 560 | Very dry surface |
| 6 | 2.3 | 130 | Very dry surface |
| 7 | 1.9 | 90 | Very dry surface |

As can be seen from Table 1, the element comprising Cellulosic Material 1 demonstrated surface tackiness whereas the others did not. Maximum swelling was demonstrated by the discs of formulations comprising Cellulosic Materials 1, 2 or 5. Discs formed from the formulation including Cellulosic Material 1 also demonstrated adherence to a mucous membrane as in the buccal cavity.

Example 2

The swelling behaviour of discs made from formulations comprising materials in the proportions shown in Table 2 was studied.

### TABLE 2

| Component | Formulation | | | | | |
|---|---|---|---|---|---|---|
| | 1 | 2 | 3 | 4 | 5 | 6 |
| Silicone Part 1A | 62.5 | 50 | 45.85 | 41.7 | 37.5 | 33.3 |
| Silicone Part 1B | 12.5 | 10 | 9.15 | 8.3 | 7.5 | 6.7 |
| Glycerol | 0 | 15 | 20 | 25 | 30 | 35 |
| Cellulosic material 1 | 25 | 25 | 25 | 25 | 25 | 25 |

A formulation containing 40% glycerol and 25% glycerol needed 15 minutes at 100°C to be fully cured. When glycerol weight % was increased to 50% curing did not take place. The sample discs were allowed to swell for 48 hours in deionized water (pH = 6) at 20°C. The results are given in Table 3.

### TABLE 3

| Formulation | Ws/Wd | Remarks |
|---|---|---|
| 1 | 1.7 | Very dry surface |
| 2 | 6.3 | Slight tack gel like surface |
| 3 | 7.2 | Tacky, gluey surface |
| 4 | 8.3 | Tacky, gluey surface |
| 5 | 2.3 | Very gluey surface |
| 6 | 1.1 | Decrease in volume |

As can be seen, presence of glycerol in the formulation provides a dramatic increase in swelling and bioadhesion. These results also show that the proportion of glycerol employed can be optimized in order to get desired swelling and good bioadhesion. Maximum swelling was obtained using 25% glycerol. At glycerol levels of 35 and 40% a decrease in volume of the disc is observed due to loss of integrity of the matrix after maximum swelling.

The swelling and bioadhesion behaviour was examined of samples made from formulations using proportions of material as shown in Table 4. Discs were prepared and swelled for 48 hours in deionized water (pH = 6) at 20°C. The results are reported in Table 5.

### TABLE 4

| Component | Formulation | | | |
|---|---|---|---|---|
| | 7 | 8 | 9 | 10 |
| Silicone Part 1A | 70.8 | 62.5 | 50.0 | 29.4 |
| Silicone Part 1B | 14.2 | 12.5 | 10 | 5.8 |
| Glycerol | 15 | 15 | 15 | 15 |
| Cellulosic material 1 | 0 | 10 | 25 | 50 |

TABLE 5

| Formulation | Ws/Wd | W% | Remarks |
|---|---|---|---|
| 7 | 1.8 | 80 | Very dry surface |
| 8 | 2.1 | 110 | Very slight tack |
| 9 | 6.3 | 530 | Slight tack |
| 10 | 5.3 | 430 | Gel like surface |

The results show that for a constant glycerol content a minimum proportion of cellulosic material is necessary in order to get significant swelling and tack. At 50% cellulosic polymer (which represents only 35% silicone in the composition) cohesion of the swelled material is poor, which results in an erosion of the surface of the disc and loss of the cellulosic material into the solution.

The swelling behaviour of sample discs made using 50 parts of Silicone Part 1A, 10 parts of Silicone Part 1B, 15 parts glycerol and 25 parts Cellulosic Material 1 was examined. Sample discs were allowed to swell over 48 hours at 20°C at eight different pH : pH = 1.2, 2, 3, 4, 5, 6, 7 and 8. The Weight Swelling Ratio, Ws/Wd, was recorded every hour during the first eight hours and then after 24 hours, 48 hours and every day up to 6 days. The results are reported in Table 6 and Table 6(A).

TABLE 6

| (H) | pH1.2 | pH 2 | pH 3 | pH 4 |
|---|---|---|---|---|
| 0 | 1.000 | 1.000 | 1.000 | 1.000 |
| 1 | 1.101 | 1.134 | 1.208 | 1.277 |
| 2 | 1.135 | 1.184 | 1.293 | 1.438 |
| 3 | 1.173 | 1.229 | 1.389 | 1.619 |
| 4 | 1.204 | 1.274 | 1.468 | 1.787 |
| 5 | 1.235 | 1.310 | 1.540 | 1.959 |
| 6 | 1.264 | 1.349 | 1.605 | 2.127 |
| 7 | 1.291 | 1.385 | 1.677 | 2.293 |
| 8 | 1.314 | 1.416 | 1.742 | 2.437 |
| 24 | 1.607 | 1.852 | 2.548 | 3.656 |
| 32 | 1.704 | 2.019 | 2.772 | 4.123 |
| 48 | 1.821 | 2.243 | 3.101 | 4.663 |
| 72 | 1.995 | 2.536 | 3.448 | 5.07 |
| 144 | 2.19 | 3.198 | 3.933 | 5.228 |

## TABLE 6(A)

| (H) | pH 5 | pH 6 | pH 7 | pH 8 |
|---|---|---|---|---|
| 0 | 1.000 | 1.000 | 1.000 | 1.000 |
| 1 | 1.290 | 1.310 | 1.297 | 1.286 |
| 2 | 1.462 | 1.466 | 1.466 | 1.434 |
| 3 | 1.643 | 1.652 | 1.648 | 1.601 |
| 4 | 1.827 | 1.816 | 1.817 | 1.738 |
| 5 | 2.011 | 2.002 | 1.988 | 1.899 |
| 6 | 2.176 | 2.178 | 2.149 | 2.049 |
| 7 | 2.346 | 2.344 | 2.311 | 2.205 |
| 8 | 2.489 | 2.500 | 2.444 | 2.346 |
| 24 | 3.682 | 3.813 | 2.644 | 3.610 |
| 32 | 4.114 | 4.218 | 4.033 | 4.020 |
| 48 | 4.668 | 4.920 | 4.570 | 4.579 |
| 72 | 5.04 | 5.266 | 4.918 | 4.921 |
| 144 | 5.076 | 5.262 | 4.976 | 4.961 |

For all pH values swelling is linear until the 8th hour. At any time, maximum swelling was obtained for pH between 4 and 7 (Ws/Wd = 2.4 to 2.5 after 8 hours) much superior than at very acidic pH (Ws/Wd = 1.3 at pH = 1.2 after 8 hours).

Example 3

Discs were prepared from formulations containing components in amounts set forth in Tables 7 and 8.

## TABLE 7

| Component | Formulation | | | | | |
|---|---|---|---|---|---|---|
| | 1 | 2 | 3 | 4 | 5 | 6 |
| Silicone Part 1A | 41.25 | 37.5 | 33.75 | 30 | 26.25 | 22.5 |
| Silicone Part 1B | 13.75 | 12.5 | 11.25 | 10 | 8.75 | 7.5 |
| Glycerol | 15 | 15 | 15 | 15 | 15 | 15 |
| Cellulosic material 1 | 25 | 25 | 25 | 25 | 25 | 25 |
| Sodium acetate | 5 | 10 | 15 | 20 | 25 | 30 |

## TABLE 8

| Component | Formulation | | | | | |
|---|---|---|---|---|---|---|
| | 7 | 8 | 9 | 10 | 1 | 12 |
| Silicone Part 1A | 45 | 41.25 | 37.5 | 33.75 | 30 | 26.25 |
| Silicone Part 1B | 15 | 13.75 | 12.5 | 11.25 | 10 | 8.75 |
| Glycerol | 15 | 15 | 15 | 15 | 15 | 15 |
| Cellulosic material 1 | 5 | 10 | 5 | 20 | 25 | 30 |
| Sodium acetate | 20 | 20 | 20 | 20 | 20 | 20 |

The discs were allowed to swell in gastric medium of pH 1.2 at room temperature. It was found that swelling occurred to an extent greater than was possible at pH 1.2 in absence of the sodium acetate. Proportions of 15, 20 and 25% of the salt promote continued swelling of the discs to a substantial extent during 48 hours. It was also found that the proportion of cellulosic material employed strongly influenced the swelling performance, larger proportions serving to promote rapid swelling to two or three times the initial size.

## Example 4

Formulations were prepared by mixing ingredients in the proportions shown in Table 9(A) and 9(B).

## TABLE 9(A)

| Component | Formulation | | | | |
|---|---|---|---|---|---|
| | A | B | C | D | E |
| Silicone Part 1A | 30 | 30 | 50 | 50 | 30 |
| Silicone Part 1B | 10 | 10 | - | - | 10 |
| Silicone Part 2B | - | - | 10 | 10 | - |
| Propylene glycol | 15 | - | 15 | - | - |
| Polyethylene glycol 200 | - | - | - | - | 15 |
| Polyethylene glycol 400 | - | - | - | - | - |
| Glycerol | - | - | - | - | |
| Sorbitol | - | 15 | - | 15 | |
| Cellulosic material 1 | 25 | 25 | 25 | 25 | 25 |
| Sodium acetate | 20 | 20 | - | - | 20 |
| Cure time at 100°C | 9'30" | 11' | 1'30" | 65" | * |
| Cure time at 18±2°C | >3H | >3H | 20' | 3' | >6H |

TABLE 9(B)

| Component | Formulation | | | | |
|---|---|---|---|---|---|
| | F | G | H | $T_1$ | $T_2$ |
| Silicone Part 1A | 30 | 50 | 50 | 30 | 50 |
| Silicone Part 1B | 10 | - | - | 10 | - |
| Silicone Part 2B | - | 10 | 10 | - | 10 |
| Propylene glycol | - | - | - | - | - |
| Polyethylene glycol 200 | - | 15 | - | - | - |
| Polyethylene glycol 400 | 15 | - | 15 | - | - |
| Glycerol | - | - | 15 | 15 | |
| Sorbitol | - | - | - | | |
| Cellulosic material 1 | 25 | 25 | 25 | 25 | 25 |
| Sodium acetate 20 | - | 20 | - | | |
| Cure time at 100°C | * | 6' | 9'30" | 9' | 1' |
| Cure time at Room Temperature | >6H | >2H | >24H | >1H | 8' |

\* Cure inhibited

The time required for cure of these compositions is dependent upon the polyol used and presence or absence of sodium acetate as well as on the nature of the Silicone Part B.

Formulations E and F which included both sodium acetate and the polyethylene glycols failed to cure properly. The others containing 20% sodium acetate required several hours to cure at room temperature. In general, the shorter cure times at room temperature are achieved with formulations using glycerol or sorbitol. Swelling of discs prepared from these formulations in 200ml distilled water or 200ml of a gastric medium of pH 1.2 or a solution of pH 7 at room temperature was measured and recorded. It was found that the discs of formulations C, D, G, H and $T_2$ swelled less in medium of pH 1.2 than they did in water. In water, formulations D, G, H, and $T_2$ swelled in a substantially constant fashion over 24 hours to a Weight Swelling Ratio in the range 1.6 to 5, with formulation $T_2$ showing the most constant swelling, and the degree of swelling of the formulations being in the order G>$T_2$>H. Formulation C (which used propylene glycol) swelled at the same rate as formulation G for 8 hours and then showed a slower swelling. In the medium of pH 1.2 the formulations swelled quickly to a Weight Swelling Ratio of about 1.1 to 1.2 and then swelled at a constant rate to a Weight Swelling Ratio in the range 1.35 to 1.65, with formulation D swelling the least and G the most. The discs of formulations A, E and $T_1$ swelled quickly in water and in the medium of pH 1.2 to double their size within 1 to 3 hours. Formulation B, after attaining double its initial size after 3 hours in water or 5 hours at pH 1.2, ceased to swell whereas the discs of formulations A, E and $T_1$ continued to swell during 24 hours. Formulation $T_1$ swelled at a substantially constant rate throughout the 24 hour period whereas formulations A and E swelled less rapidly after 8 hours immersion.

Example 5

Films were made by pressing formulations between polyester films in a mould for 10 minutes at 50°C using formulations comprising components in the proportions shown in Table 10. In these formulations the proportion of silicone polymer employed is varied, whilst maintaining fixed the ratios of cellulosic material to the other non-silicone components (ratio 5/12) of sodium acetate to other non-silicone components (1/3) and the ratio of glycerol to other non-silicone components (1/4) and using the same proportion of barium sulphate.

TABLE 10

Formulation

| Component | 1 | 2 | 3 | 4 | 5 | 6 |
|---|---|---|---|---|---|---|
| Silicone Part 1A | 30 | 33.75 | 37.51 | 41.25 | 45 | 48.75 |
| Silicone Part 1B | 10 | 11.25 | 12.51 | 13.75 | 15 | 16.25 |
| Glycerol | 13.75 | 12.5 | 11.25 | 10.1 | 8.75 | 7.51 |
| Cellulosic material 1 | 22.92 | 20.83 | 18.75 | 16.67 | 14.58 | 12.5 |
| Sodium acetate | 18.33 | 16.67 | 15.1 | 13.33 | 11.67 | 10 |
| Barium sulphate | 5 | 5 | 5 | 5 | 5 | 5 |

Swelling of these films at 37°C in gastric medium (pH 1.2) was examined. The films from formulations including smaller proportions of silicone swelled more quickly and to a greater extent than those with greater proportions of silicone, those containing 40% and 45% silicone respectively swelling to at least 1.75 times their original size within 2 hours. The same formulations were moulded as rings 2mm thick (circumference of the solid 147mm). The rings from formulations including smaller proportions of silicone swelled more quickly than those with greater proportions of silicone. The rings containing 40, 45 and 50% silicone doubled their size within 2 hours and continued to swell to at least 3.5 times their original size within 8 hours; thereafter they continued to swell until after about 24 hours they began to shrink. Rings from formulations containing 60 and 65% silicone swelled to 1.5 times their original size within 2 hours and continued to swell over 48 hours to at least 2.5 times their original size.

Example 6

Particulate elements were made in a Reocord 40 granulating mixer machine. In one procedure, formulations were used comprising materials in the amounts shown in Table 11.

TABLE 11

Formulation

| Component | 1 | 2 | 3 | 4 | 5 | 6 |
|---|---|---|---|---|---|---|
| Aspirin | 45 | 40 | 25 | 45 | 40 | 25 |
| Silicone Part 1A | 3.75 | 7.5 | 18.75 | 3.75 | 7.5 | 18.75 |
| Silicone Part 1B | 1.25 | 2.5 | 6.25 | 1.25 | 2.5 | 6.25 |
| Process fluid | 6 | 6 | 6 | - | - | - |

The process fluid used was pharmaceutical grade hexamethyldisiloxane. The machine was set to granulate the formulations 1 to 6 with blades operating at 200 rpm. The temperature rose from about 22°C to about 45°C. Cured particles were formed within 10 minutes in each case and the particles were of a substantially uniform size in the range 200 to 1000 micrometers. The particles were separated into desired size ranges by sieving. In a second procedure, particles were formed as above using a formulation comprising 10.5 parts aspirin, 23.3 parts silicone Part 1A and 7.65 parts silicone Part 1B. The particles were recovered, sieved and calibrated. Particles of selected size were returned to the granulating machine together with 22.3 parts silicone Part 1A and 7.45 parts silicone Part 1B. The particles were thus coated with a cured silicone. The coated particles were recovered, sieved and calibrated.

When equal parts of Silicone Parts M1A and M1B were dry granulated at room temperature, particles of a well cured, rigid foam of density 0.13 g/cc were formed within 6 minutes. When granulated with 30% dicalcium phosphate, granules were formed which floated well in gastric medium of pH 1.2. 1.5 parts of silicone Parts M1A and M1B in a ratio 1:1 were granulated with 5 parts of a mixture of 90% calcium phosphate and 10% sorbitol at room temperature for 5 minutes. The granules produced floated spontaneously in the solution of pH 1.2. They were found to be well cured, rigid particles having a size in the range 250 to 1000 micrometers and having cells up to about 300 micrometers.

Example 7

Two physical forms were prepared using a formulation comprising components in proportions shown in Table 12.

TABLE 12

| Component | Formulation |
|---|---|
| Silicone Part 2A | 30 |
| Silicone Part 1B | 10 |
| Glycerol | 13.75 |
| Cellulosic material 1 | 22.92 |
| Sodium acetate | 18.33 |
| Barium sulphate | 5 |

The cellulosic material, sodium acetate and barium sulphate were mixed in a Waring Blender. 23.13 parts of this mixture were placed in the Rheocord system 40 granulating machine and mixed for 2 minutes. The silicone Part 1B was added to the mixture and the granulator operated with its mixing chamber at 30°C with the blades operating at 190 rpm for 25 minutes. The Silicone Part 2A and the glycerol were mixed by hand and then mixed with the wetted powder in the ratio 5.63 parts wetted powder to 4.37 parts Part 2A plus glycerol using a Bioblock Scientific mixer. Lead particles were incorporated into the mixture and a portion of the mixture was placed in a press between two foils of Mylar 125 micrometers thick and cured for 10 minutes at 50°C or for 20 minutes at room temperature to form a film 1mm thick. Strips 2cm x 3cm were cut from this film. The strips weighed about 0.686g. They were placed in gelatin capsules, size '0' of average weight 109mg.

A portion of the mixture taken from the Bioblock Scientific mixer and including lead particles was injected by means of a syringe into moulds 7.8 mm in diameter and 6cm long. The mixture in the moulds was heated to 50°C for 10 minutes. Once the formulation had cured and cooled, each moulding was cut to provide a cylindrical element 2cm long and 7.5mm in diameter having an average weight of 1. 198g.

The capsules and the cylindrical elements were administered orally to four dogs in a cross over study. Each dog had fasted for 24 hours prior to the study and received a standardised meal together with 50 ml water and the capsules or elements. During the study, the dogs received a standardised meal every day and water ad libidum. The progression of the dosage forms was evaluated by X-Ray scanning at time intervals of 0, 2, 4, 8 and 24 hours. The dosage forms were detected in the stomach up to and including the 8th hour in all the dogs for both dosage forms. The X-Ray images showed the cylindrical elements swelled to double their initial size in the stomachs of the dogs. As far as the rolled up strips were concerned, the X-Ray images showed the strips uncoiling after two hours and swelling of the strips was clearly noticeable. The elements of the dosage forms did not disintegrate in the dog but were discharged as such. When recovered from the faeces the elements were found to demonstrate a good similarity in terms of in-vitro/in-vivo swelling behaviour.

Example 8

Release from a swellable element of the drug Theophylline was examined. Theophylline is characterised by a uniform absorption throughout all the gastrointestinal tract. An illustrative formulation was prepared from 40 parts silicone Part 1A, 8 parts silicone Part 1B (i.e. 48 parts of the curable silicone), 12 parts of glycerol, 20 parts of Cellulosic Material 1 and 20 parts of Theophylline. The cellulosic material and the Theophylline were mixed with glycerol then incorporated into the curable silicone. A comparative formulation was prepared in the same way comprising 68 parts of the same curable silicone, 12 parts glycerol, 20 parts Theophylline but no cellulosic material. Both compositions were pressed into an aluminium mould between two polyester films and cured at 100°C for 10 minutes to provide matrices 2.25mm thick. 1cm diameter discs (around 220mg each) were punched out from the matrices and their weight recorded. Release of the drug from the elements was examined by Dissolution study at pH 1.2. and 4.5 according to the USP XXXI Standard test and the basket method using a fully automatic SOTAX AT6, including the dissolution apparatus itself, linked to a microprocessor pneumatic pump and fraction collector. Six dissolution vessels were filled with 900ml of dissolution medium. One disc was placed in each basket using three discs of each formulation. Stirring was effected at 150 rpm with the outer thermostatic bath at 37 ± 0.1°C. 5ml samples of dissolution medium were collected at preprogrammed time intervals with automatic replenishment with fresh medium. UV spectra of these samples were recorded with a UVIKON 860 spectrophotometer and Theophylline content calculated from the standard curve. The mean value of the cumulative amount as a percentage of Theophylline released from the six cells for each pH value is shown in Tables 13 and 14.

TABLE 13

Cumulative % Theophylline released as a function of time at pH = 1.2

| Time Hours | Illustrative Formulation | Comparative Formulation |
|---|---|---|
| 0 | 0.00 | 0.00 |
| 0.5 | 5.66 | 2.06 |
| 1 | 8.77 | 2.42 |
| 1.5 | 11.54 | 2.64 |
| 2 | 14.14 | 2.87 |
| 3 | 18.91 | 3.16 |
| 4 | 23.50 | 3.51 |
| 5 | 28.09 | 3.77 |
| 6 | 32.81 | 4.08 |
| 7 | 37.30 | 4.25 |
| 8 | 42.40 | 4.61 |
| 12 | 56.30 | 5.47 |
| 18 | 71.65 | 6.64 |
| 24 | 82.51 | 7.66 |

## TABLE 14

### Cumulative % Theophylline released as a function of time at pH = 4.5

| Time Hours | Illustrative Formulation | Comparative Formulation |
|---|---|---|
| 0 | 0.00 | 0.00 |
| 0.5 | 5.90 | 1.99 |
| 1 | 10.68 | 2.54 |
| 1.5 | 15.40 | 2.78 |
| 2 | 20.15 | 3.03 |
| 3 | 29.94 | 3.53 |
| 4 | 39.34 | 3.92 |
| 5 | 48.22 | 4.39 |
| 6 | 56.11 | 4.72 |
| 7 | 63.11 | 5.06 |
| 8 | 69.39 | 5.40 |
| 12 | 88.00 | 6.63 |
| 18 | 94.66 | 8.04 |
| 24 | 95.08 | 9.37 |

The results demonstrate three important points:
- The presence of cellulosic material is essential to allow substantial release of Theophylline from the silicone matrix. In the absence of the hydrophilic polymer, maximum drug release after 24 hours is less than 10% at either pH.
- The release of Theophylline is very limited when swelling is negligible as shown by comparison of release values at pH = 4.5 versus pH = 1.2.
- Zero order release is achieved when cellulosic material is used. This can be verified from linear regression on % drug release = f (time), which gives the following results for the correlation coefficient r:

```
At pH = 1.2   r = 0.9990 between 0.5h and  8h (10 points)
At pH = 4.5   r = 0.9987 between 0    and  6h ( 9 points)
              r = 0.9953 between 0    and  8h (11 points)
              r = 0.9740 between 0    and 12h (12 points)
```

A swelling study was conducted on drug free discs in the conditions used for dissolution. 1cm diameter discs were made from 60 parts of the same curable silicone, 15 parts glycerol and 25 parts cellulosic material. The dissolution apparatus was used, one disc was placed in each basket at 37°C. Swelling was recorded for both pH by weight at regular intervals. Table 15 reports the Weight Swelling Ratio Ws/Wd along with the corresponding values of % Theophylline released (from Tables 13 and 14) for pH = 1.2 and 4.5.

## TABLE 15

| Time (H) | WS/WD pH 1.2 | % Released pH 1.2 | Ws/Wd pH 4.5 | % Released pH 4.5 |
|---|---|---|---|---|
| 0 | 1 | 0 | 1 | 0 |
| 1 | 1.246 | 8.77 | 1.430 | 10.68 |
| 2 | 1.319 | 14.14 | 1.703 | 20.15 |
| 3 | 1.379 | 18.91 | 1.967 | 29.94 |
| 4 | 1.430 | 23.50 | 2.196 | 39.34 |
| 5 | 1.475 | 28.08 | 2.432 | 48.22 |
| 6 | 1.520 | 32.81 | 2.686 | 56.11 |
| 7 | 1.557 | 37.30 | 2.875 | 63.11 |
| 8 | 1.590 | 42.40 | 3.099 | 69.39 |
| 24 | 1.963 | 82.51 | 5.094 | 95.08 |

Under dissolution conditions results are consistent with what was observed in the swelling tests of previous Examples. Thus, using discs made from formulations containing no cellulosic material, swelling is very low and not pH dependent (Ws/Wd = 1.5 at pH 1.2, Ws/Wd = 1.7 at pH 4.5 after 24 hours), whereas with discs made from formulations including cellulosic material, a high degree of swelling is obtained only at pH 4.5 (Ws/Wd = 1.9 at pH 1.2, Ws/Wd = 5.1 at pH 4.5 after 24 hours). From Table 15 it can be seen that there is an at least substantially linear relationship between % drug release and polymer swelling which is especially true at pH = 4.5

Example 9

A formulation was prepared comprising 37.5 parts of silicone Part 2A and 25 parts of silicone Part 2B, 9 parts of sorbitol, 19.5 parts of glycerol and 9 parts of the drug tetracycline hydrocholoride. Matrices were formed from this composition by moulding at room temperature. The matrices weighed 961.3mg, had an average thickness of 0.66cm and a surface area of about 5.2cm$^2$. Release of the drug from the silicone composition was examined by dissolution study as referred to in Example 8. One matrix was placed in each basket of the dissolution apparatus. Stirring was effected at 150 rpm with the outer thermostatic bath at 37 +/- 0.1°C. 5ml samples were collected at preprogrammed time intervals with automatic replenishment with fresh medium. UV spectra were recorded with the UVIKON 860 spectrophotometer and tetracycline hydrochloride content calculated from the calibration curve. The mean value of the cumulative amount, as a percentage, of tetracycline hydrochloride released from the six cells is shown in Table 16.

## TABLE 16

| Time (Hrs) | Mean Value (%) |
|---|---|
| 0 | 0 |
| 0.5 | 1.30 |
| 1 | 1.70 |
| 2 | 2.36 |
| 3 | 2.85 |
| 4 | 3.24 |
| 5 | 3.56 |
| 6 | 3.97 |
| 12 | 5.80 |
| 18 | 7.68 |
| 24 | 9.62 |
| 36 | 14.84 |
| 48 | 18.57 |
| 60 | 22.98 |
| 72 | 27.01 |
| 84 | 30.52 |
| 96 | 34.46 |
| 108 | 38.57 |
| 120 | 41.95 |
| 132 | 46.02 |
| 144 | 48.88 |
| 156 | 52.38 |
| 168 | 55.33 |
| 180 | 58.51 |
| 192 | 60.44 |
| 204 | 67.59 |
| 216 | 68.64 |
| 228 | 69.56 |
| 240 | 70.20 |

These results show that about 0.317% tetracycline hydrochloride was released per hour and 7.6% was released per day during dissolution. The release occurred at substantially constant rate over the whole test period. The proportion liberated attained a plateau after 8.5 days and 70.2% of the drug had been released at the ninth day. At the end of the dissolution test the mean surface area of the matrices was 8.7cm$^2$ i.e. 1.7 times the original surface area.

Example 10

A first formulation was prepared from 15 parts silicone Part 2A, 3 parts silicone Part 2B, 4.5 parts glycerol, 7.5 parts cellulosic material 1 and 70 parts of aspirin. A second formulation was prepared from 21.26 parts silicone Part 2A, 4.24 parts silicone Part 2B and 4.5 parts glycerol. The two formulations were granulated using the Rheocord 40 granulating mixer. The machine was set to granulate the formulations with blades rotating at 200 rpm. The temperature rose from about 22°C to about 45°C. Cured particles of a substantially uniform size in the range 200 to 1000 micrometers were formed within 10 minutes. Particles in the size range 400 to 630 micrometers were separated by sieving and these particles were subjected to the dissolution test referred to in Example 8. In the test, the particles were placed in medium of pH 4.5, stirring was effected at 100 rpm and the bath was maintained at 37°C. The elements of both formulations liberated the aspirin and both delivered about 40% of the aspirin during the first hour. The formulation without cellulosic material then proceeded to deliver the drug at a progressively reducing rate, with 60% of the drug being released within 8 hours and 74% of the aspirin being released during 24 hours. The formulation with cellulosic material 1 continued to deliver the drug at a high rate, 85% of the aspirin being released during the first five hours and 92% during the first 8 hours.

## Claims

1.  A sustained release element comprising an agent (A) capable of release from the element when the element is in an aqueous medium, a cured silicone material and a hydrophilic organic material characterised in that the element consists of a vehicle (B) comprising a resilient silicone polymer body having mixed therein the agent (A), the polymer body being formed from a curable composition comprising a polysiloxane containing alkyl hydrogen siloxane units, a polysiloxane containing unsaturated groups for reaction therewith, and a platinum or rhodium catalyst, 5 to 40% by weight of the vehicle of a hydrophilic component and up to 40% by weight of the vehicle of a modulating component which serves to modulate the release of agent (A) selected from (I) an organic hydrophilic substance having two or more hydroxyl groups per molecule and/or (II) an organic hydrophilic polymer which swells in an aqueous medium.

2.  An element according to Claim 1 further characterised in that the hydrophilic component is selected from the group consisting of propylene glycol, liquid polyethylene glycols and glycerol and is present to an extent of 10% to 30% by weight of the vehicle.

3.  An element according to Claim 1 further characterised in that the organic substance (I) is selected from the group consisting of polyethylene glycols of molecular weight greater than 1500, sorbitol, manitol and lactose and is present to an extent of from 5% to 25% by weight of the vehicle.

4.  An element according to Claim 1 further characterised in that the hydrophylic polymer (II) is selected from the group consisting of hydroxypropyl cellulose, carboxymethyl cellulose and sodium carboxy methyl cellulose whether crosslinked or not and is present to an extent of up to 40% by weight of the vehicle.

5.  An element according to Claim 1 further characterised in that the vehicle comprises up to 30% by weight of a buffer salt effective to permit swelling of the hydrophilic polymer (II) in aqueous medium of pH 1 to 4.

6.  An element according to any one of the preceding claims further characterised in that the vehicle comprises an organic material which contributes to bioadhesive properties of the element.

7.  An element according to any one of the preceding claims further characterised in that the element is in the form of a particle, microcapsule, film, rod, ring, pad, patch, or matrix for administration to the human or animal body.

8.  An element according to any one of the preceding claims for use in a therapeutic or diagnostic method characterised in that the agent (A) is a therapeutic or diagnostic agent and is selected from antibiotic, antiseptic, antiinflammatory, cardiovascular, antiH$_2$, bronchodilator, analgesic, antiarrythmic, antihistamine, $\alpha$-1 blocker, beta blocker, ACE inhibitor, diuretic, antiaggregant, sedative, tranquiliser, anticonvulsant and anticoagulant agents, vitamins, agents for treating gastric and duodenal ulcers, proteolytic enzymes, healing factors and peptides.

9.  An element according to Claim 1 in a form suitable for oral administration comprising the components (A) and (B) in a proportion of from 0.5 to 95, preferably up to 50, parts by weight of the agent (A) and from 99.50 to 5 parts by weight of the vehicle (B), the vehicle (B) comprising 10 to 35% by weight of the hydrophilic component, 10 to 60% by weight of the silicone polymer and 10 to 40% by weight of a modulating component selected from hydroxypropyl cellulose, carboxymethyl cellulose or sodium carboxy methyl cellulose whether crosslinked or not and mixtures thereof.

10. An element according to Claim 1 in a form suitable for administration to a cavity of the body comprising the components (A) and (B) in a proportion of from 0.5 to 80, preferably 40 to 60, parts by weight of the agent (A) and from 99.5 to 40 parts by weight of the vehicle (B), the vehicle (B) comprising 10 to 40% by weight of the hydrophilic component, 40 to 80% by weight of the silicone polymer and 10 to 40% by weight of a modulating component selected from polyethylene glycols of molecular weight greater than 1500, sorbitol, manitol and lactose and mixtures thereof.

11. A method of making an element according to any one of the preceding claims which comprises mixing the agent (A) and a formulation which is curable to provide the vehicle (B) therefor comprising 5 to 40% by weight of the vehicle of the hydrophilic component and 10 to 40% by weight of the vehicle of the modulating component and a platinum or rhodium catalysed curable silicone composition comprising a polysiloxane having alkylhydrogen siloxane units and a polysiloxane having unsaturated groups for reaction therewith and coating, granulating, moulding, extruding or otherwise shaping the mixture as the silicone composition cures.

## Patentansprüche

1. Element mit Depotwirkung, umfassend ein Mittel (A), das aus dem Element freigesetzt werden kann, wenn das Element in wäßrigem Medium ist, ein gehärtetes Silikonmaterial und ein hydrophiles organisches Material, dadurch gekennzeichnet, daß das Element aus einem Träger (B) besteht, der einen elastischen Silikonpolymerkörper, dem das Mittel (A) zugemischt wurde, wobei der Polymerkörper aus einer härtbaren Zusammensetzung gebildet wird, die ein Polysiloxan, das Alkylhydrogensiloxaneinheiten enthält, ein Polysiloxan, das ungesättigte Gruppen zur Reaktion damit enthält, und einen Platin- oder Rhodiumkatalysator, bezogen auf den Träger 5 bis 40 Gew.-%, einer hydrophilen Komponente und, bezogen auf den Träger, bis zu 40 Gew.-% einer modulierenden Komponente, die dazu dient, die Freisetzung von Mittel (A) zu modulieren, ausgewählt aus (I) einer organischen hydrophilen Substanz mit zwei oder mehr Hydroxylgruppen pro Molekül und/oder (II) einem organischen hydrophilen Polymer, das in einem wäßrigen Medium quillt, umfaßt.

2. Element nach Anspruch 1, dadurch gekennzeichnet, daß die hydrophile komponente ausgewählt ist aus der Gruppe bestehend aus Propylenglykol, flüssigen Polyethylenglykolen und Glycerin und in einem Anteil von 10 bis 30 Gew.-%, bezogen auf den Träger, vorhanden ist.

3. Element nach Anspruch 1, dadurch gekennzeichnet, daß die organische Substanz (I) ausgewählt ist aus der Gruppe bestehend aus Polyethylenglykolen mit einem Molekulargewicht von mehr als 1500, Sorbit, Mannit und Lactose und in einem Anteil von 5 bis 25 Gew.-%, bezogen auf den Träger, vorhanden ist.

4. Element nach Anspruch 1, dadurch gekennzeichnet, daß das hydrophile Polymer (II) ausgewählt ist aus der Gruppe bestehend aus Hydroxypropylcellulose, Carboxymethylcellulose und Natriumcarboxymethylcellulose, vernetzt oder nicht vernetzt, und in einem Anteil von bis zu 40 Gew.-%, bezogen auf den Träger, vorhanden ist.

5. Element nach Anspruch 1, dadurch gekennzeichnet, daß der Träger bis zu 30 Gew.-% eines Puffersalzes umfaßt, das wirksam ist, um ein Quellen des hydrophilen Polymers (II) in wäßrigem Medium mit pH 1 bis 4 zuzulassen.

6. Element nach einem der vorhergehenden Ansprüche, dadurch gekennzeichnet, daß der Träger ein organisches Material umfaßt, das dem Element bioadhäsive Eigenschaften vermittelt.

7. Element nach einem der vorhergehenden Ansprüche, dadurch gekennzeichnet, daß das Element in Form eines Teilchens, einer Mikrokapsel, eines Films, Stabes, Rings, Kissens, Pflasters oder einer Matrix zur Verabreichung an den menschlichen oder tierischen Körper ist.

8. Element nach einem der vorhergehenden Ansprüche zur Verwendung in einem therapeutischen oder diagnostischen Verfahren, dadurch gekennzeichnet, daß das Mittel (A) ein therapeutisches oder diagnostisches Mittel ist und ausgewählt ist aus Antibiotika, Antiseptika, entzündungshemmenden Mitteln, kardiovaskulären Mitteln, Anti-$H_2$-Mitteln, bronchienerweiternden Mitteln, antiarrythmischen Mitteln, Analgetika, Antihistaminika, $\alpha$-1-Blockern, ß-Blockern, ACE-Inhibitoren, Diuretika, antiaggregierenden Mitteln, Sedativa, Tranquilizern, krampfhemmenden Mitteln und gerinnungshemmenden Mitteln, Vitaminen, Mitteln zur Behandlung von Magen- und Zwölffingerdarmgeschwüren, proteolytischen Enzymen, Heilfaktoren und Peptiden.

9. Element nach Anspruch 1 in einer Form, die geeignet ist zur oralen Verabreichung, umfassend die Komponenten (A) und (B) in einem Verhältnis von 0,5 bis 95, vorzugsweise bis zu 50, Gew.-Teilen Mittel (A) zu 99,50 bis 5 Gew.-Teilen des Trägers (B), wobei der Träger (B) 10 bis 35 Gew.-% hydrophile Komponente, 10 bis 60 Gew.-% Silikonpolymer und 10 bis 40 Gew.-% einer modulierenden Komponente ausgewählt aus Hydroxypropylcellulose, Carboxymethylcellulose oder Natriumcarboxymethylcellulose, vernetzt oder nicht, und Mischungen davon, umfaßt.

10. Element nach Anspruch 1 in einer Form, die geeignet ist zur Verabreichung in eine Körperhöhle, umfassend die Komponenten (A) und (B) in einem Verhältnis von 0,5 bis 80, vorzugsweise 40 bis 60, Gew.-Teilen des Mittels (A) zu 99,5 bis 40 Gew.-Teilen des Trägers (B), wobei der Träger (B) 10 bis 40 Gew.-% hydrophile Komponente, 40 bis 80 Gew.-% Silikonpolymer und 10 bis 40 Gew.-% einer modulierenden Komponente ausgewählt aus Polyethylenglykolen mit einem Molekulargewicht von mehr als 1500, Sorbit, Mannit und Lactose und Mischungen davon umfaßt.

11. Verfahren zur Herstellung eines Elementes nach einem der vorhergehenden Ansprüche, umfassend, daß man das Mittel (A) und eine Formulierung, die zu dem Träger (B) härtbar ist, die 5 bis 40 Gew.-%, bezogen auf den Träger, hydrophile Komponente und 10 bis 40 Gew.-%, bezogen auf den Träger, der modulierenden Komponente und eine durch Platin oder Rhodium katalysierte härtbare Silikonzusammensetzung, die ein Polysiloxan mit Alkylhydrogensiloxaneinheiten und ein Polysiloxan mit ungesättigten Gruppen zur Reaktion damit umfaßt, vermischt und die Mischung beschichtet, granuliert, formt, extrudiert oder in anderer Weise formt, wenn die Silikonzusammensetzung härtet.

## Revendications

1. Elément à libération prolongée comprenant un agent (A) pouvant être libéré de l'élément lorsque celui-ci se trouve dans un milieu aqueux, une matière silicone durcie et une matière organique hydrophile, caractérisé en ce que l'élément consiste en un véhicule (B) comprenant un corps en polymère de silicone élastique dans lequel est mélangé l'agent (A), le corps en polymère étant formé d'une composition durcissable comprenant un polysiloxane contenant des motifs alkylhydrogénosiloxanes, un polysiloxane contenant des groupes insaturés pour réaction avec celui-ci, et un catalyseur au platine ou au rhodium, 5 à 40 % en poids du véhicule étant constitués par un composant hydrophile et jusqu'à 40 % en poids du véhicule étant constitués d'un composant modulateur qui sert à moduler la libération de l'agent (A) et est choisi parmi (I) une substance organique hydrophile ayant deux ou plusieurs groupes hydroxyle par molécule et/ou (II) un polymère organique hydrophile qui gonfle dans un milieu aqueux.

2. Elément selon la revendication 1, caractérisé, en outre, en ce que le composant hydrophile est choisi parmi le propylène-glycol, les polyéthylèneglycols liquides et le glycérol, et est présent en une porportion de 10% à 30% en poids du véhicule.

3. Elément selon la revendication 1, caractérisé, en outre, en ce que la substance organique (I) est choisie parmi les polyéthylène-glycols de poids moléculaire supérieur à 1500, le sorbitol, le mannitol et le lactose, et est présente en une proportion de 5% à 25% en poids du véhicule.

4. Elément selon la revendication 1, caractérisé, en outre, en ce que le polymère hydrophile (II) est choisi parmi l'hydroxypropyl-cellulose, la carboxyméthyl-cellulose et la carboxyméthyl-cellulose sodique réticulée ou non, et est présent en une proportion de jusqu'à 40% en poids du véhicule.

5. Elément selon la revendication 1, caractérisé, en outre, en ce que le véhicule comprend jusqu'à 30 % en poids d'un sel tampon efficace pour permettre le gonflement du polymère hydrophile (II) dans un mileu aqueux ayant un pH de 1 à 4.

6. Elément selon l'une quelconque des revendications précédentes, caractérisé, en outre, en ce que le véhicule comprend une substance organique qui contribue aux propriétés bioadhésives de l'élément.

7. Elément selon l'une quelconque des revendications précédentes, caractérisé, en outre, en ce qu'il est sous la forme d'une particule, d'une microcapsule, d'une pellicule, d'un bâtonnet, d'un anneau, d'une compresse, d'un timbre ou d'une matrice pour une administration au corps humain ou animal.

8. Elément selon l'une quelconque des revendications précédentes à utiliser dans une méthode thérapeutique ou diagnostique, caractérisé en ce que l'agent (A) est un agent thérapeutique ou diagnostique et est choisi parmi les agents antibiotiques, antiseptiques, anti-inflammatoires, cardio-vasculaires, anti-$H_2$, bronchodilatateurs, analgésiques, antiarythmiques, antihistaminiques, alpha-1-bloquants, bêtabloquants, inhibiteurs d'ECA, diurétiques, anti-agrégants plaquettaires, sédatifs, tranquillisants, anticonvulsivants et anticoagulants, les vitamines, les agents pour le traitement d'ulcères gastriques et duodénaux, les enzymes protéolytiques, les facteurs de cicatrisation et les peptides.

9. Elément slon la revendication 1, qui est sous une forme adaptée à l'administration par voie orale et comprend 0,5 à 95, de préférence jusqu'à 50, parties en poids de l'agent (A) et 99,50 à 5 parties en poids du véhicule (B), le véhicule (B) comprenant 10 à 35% en poids du composant hydrophile, 10 à 60% en poids du polymère de silicone et 10 à 40% en poids d'un composant modulateur choisi parmi l'hydroxypropyl-cellulose, la carboxyméthyl-cellulose et la carboxyméthyl-cellulose sodique réticulée ou non, et leurs mélanges.

10. Elément selon la revendication 1, qui est sous une forme adaptée à l'administration à une cavité du corps et comprend 0,5 à 80, de préférence 40 à 60, parties en poids de l'agent (A) et 99,5 à 40 parties en poids du véhicule (B), le véhicule (B) comprenant 10 à 40 % en poids du composant hydrophile, 40 à 80% en poids du polymère de silicone et 10 à 40% en poids d'un composant modulateur choisi parmi les polyéthylène-glycols de poids moléculaire supérieur à 1500, le sorbitol, le mannitol, le lactose et leurs mélanges.

11. Procédé pour fabriquer un élément selon l'une quelconque des revendications précédentes, qui consiste à mélanger l'agent (A) et une formulation qui est durcissable pour former le véhicule (B) et comprend un composant hydrophile formant 5 à 40% en poids du véhicule, un composant modulateur formant 10 à 40% en poids du véhicule et une composition de silicone durcissable catalysée par du platine ou du rhodium comprenant un polysiloxane ayant des motifs alkylhydrogénosiloxanes et un polysiloxane ayant des groupes insaturés pour réagir avec ceux-ci, et à enrober, granuler, mouler, extruder ou mettre en forme d'une autre manière le mélange tandis que la composition de silicone durcit.